(19) Europäisches Patentamt / European Patent Office / Office européen des brevets

(11) **EP 3 669 780 B1**

(12) **EUROPEAN PATENT SPECIFICATION**

(45) Date of publication and mention of the grant of the patent:
**04.10.2023 Bulletin 2023/40**

(51) International Patent Classification (IPC):
**A61B 5/12** $^{(2006.01)}$

(21) Application number: **18215560.6**

(52) Cooperative Patent Classification (CPC):
**A61B 5/123;** A61B 2560/0247

(22) Date of filing: **21.12.2018**

(54) **METHODS, DEVICES AND SYSTEM FOR A COMPENSATED HEARING TEST**

VERFAHREN, VORRICHTUNGEN UND SYSTEM FÜR EINEN KOMPENSIERTEN HÖRTEST

PROCÉDÉS, DISPOSITIFS ET SYSTÈME DE TEST AUDITIF À COMPENSATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.06.2020 Bulletin 2020/26**

(73) Proprietor: **Audiodo AB (publ)**
**211 13 Malmö (SE)**

(72) Inventors:
• **PHILIPSSON, John**
**247 35 Södra Sandby (SE)**
• **MARTINSON, Roger**
**211 16 Malmö (SE)**

(74) Representative: **Ström & Gulliksson AB**
**P.O. Box 793**
**220 07 Lund (SE)**

(56) References cited:
US-A- 6 160 893          US-A1- 2005 059 904
US-A1- 2014 309 549      US-A1- 2015 358 745
US-A1- 2018 098 720

• YOOK SUNHYUN ET AL: "Modified segmental signal-to-noise ratio reflecting spectral masking effect for evaluating the performance of hearing aid algorithms", SPEECH COMMUNICATION, vol. 55, no. 10, 19 June 2013 (2013-06-19) , pages 1003-1010, XP028692997, ISSN: 0167-6393, DOI: 10.1016/J.SPECOM.2013.05.005
• Na Youngmin ET AL: "Smartphone-Based Hearing Screening in Noisy Environments", Sensors, vol. 14, no. 6, 12 June 2014 (2014-06-12), pages 10346-10360, XP093007913, CH ISSN: 1424-8220, DOI: 10.3390/s140610346

## Description

## TECHNICAL FIELD

The present invention relates to hearing test for generating a hearing profile and particularly to hearing tests performed in an uncontrolled environment

## BACKGROUND

Since the introduction of portable electronic equipment such as the Walkman™ in the late 1970's, the availability and quality of portable audio has been ever improving. With the smartphone, every user of such a device has access to an almost endless supply of music and other audio content. The audio content will typically be experienced through a pair of headphones or other portable speakers.

The audio content is intended to sound in a specific way, e.g. the author and the producer of the content have a certain idea of how the content should be perceived by a listener. The same can be said with regards to the speakers and the headphone. The designers of those products typically have a vision of how their product should alter the audio content in order to create e.g. a brand specific sound or feeling.

The way the actual audio content is perceived by a listener will depend greatly on the hearing profile of the listener. Audio and audio playback equipment is designed for a listener having an average hearing profile or a listener having a substantially flat hearing profile. In either way, having the perceived audio compensated for the hearing profile of the listener greatly improves the listening experience.

The basic concept of compensating audio content based on a hearing profile of a user is known and described in US2006215844 which relates in general to methods and systems for filtering and amplifying audio signals to improve the listening environment for both normal and hearing-impaired persons. US2006215844 bases the filtering and amplifying of audio signals on a determined hearing profiles of a listener.

One way of performing a hearing test in order to create a hearing profile of a user is to subject the user to audio test tones of different frequencies. The user is requested to indicate if the test tone is perceived and if this is the case, the intensity of the test tone is decreased until the user is unable to perceive the test tone. The lowest intensity where the test tone was perceived by the user will, together with the frequency of test tone, constitute one data point in the hearing profile. This hearing profile may in turn be used to compensate the frequency response of audio such that the audio is compensated for any hearing impairments of the user.

The determining of the hearing profile will require a silent, or at least quiet surrounding in order to be accurate. Background noise and other forms of audio pollution will affect the hearing profile and may result in an incorrect profile.

CN101862195A relates to a circuit for use in hearing tests and assisted hearing, and more particularly to a hearing test and hearing aid system.

US 2015358745 presents an earpiece for use in conducting a hearing test, a hearing test kit and a method of conducting a hearing test. The earpiece includes a speaker which generates stimuli and a microphone which detects ambient noise. The stimuli generated by the speaker compensates for the ambient noise detected by the microphone.

US 2014309549 describes a method for testing hearing ability of a used by outputting a first audible tone including a first frequency and recording a first volume adjustment for the first audible tone by the user. Outputting a second audible tone including a second frequency and recording a second volume adjustment for the second audible tone by the user. The method further includes selecting default settings for the first and second signals based on a noise floor proximal the user.

US 6160893 pertains to an active noise control system for use in testing hearing using a pure tone audiometry testing procedure and employing multiple switching controllers with pre-filtering means.

US 2018098720 speak of a method and a set of headphones to enable a user of the set of headphones to conduct a self-administered hearing test.

In "Smartphone-Based Hearing Screening in Noisy Environments" by Youngmin et al. 2014, a smartphone automatically measures the environmental noise and provides a hearing threshold shift value to correct the hearing threshold during an ubiquitous hearing test.

The determining of the hearing profile by performing a hearing test is known. But the prior art fails to disclose how to determine a hearing profile in an uncontrolled environment with stochastic sounds in combination with background noise.

In view of the above, it is evident that there is room for improvements.

## SUMMARY

An object of the present invention is to provide a new type of hearing test which is improved over prior art and which eliminates or at least mitigates the drawbacks discussed above. Another object of the invention is to provide a hearing test performed in an uncontrolled environment from which it is possible to generate an accurate hearing profile. One or more of these objects are achieved by the technique set forth in the appended independent claims with preferred embodiments defined in the dependent claims related thereto.

According to a first aspect, a method for noise compensating a hearing test performed in an uncontrolled environment is provided. The hearing test being performed by generating one or more test tones of a number of test tone frequencies, presenting, using an audio speaker, said test tones to a user, receiving user input indicative of the users perception of the test tones such that a test result comprising intensity levels of hearing for the number of test tone frequencies is provided, and generating a hearing profile based on the test result. The method comprises evaluating a noise level received from a microphone, determining a noise perception model based on said noise level, and compensating at least one of the test result or said one or more test tones using the noise perception model.

The step of evaluating the noise perception model comprises calculating a noise power spectrum based on the evaluated noise level. The noise power spectrum comprising noise level for a number of frequency ranges. The noise power spectrum is an efficient, flexible and accurate representation of the evaluated noise level. The number of frequency ranges may be the same as the number of test tone frequencies. This allows for simple and efficient correlation between test tones and frequency ranges and opens up for bandwidth optimization etc.

The noise perception model further comprises determining off frequency masking between the one or more test tone frequencies and the noise power spectrum for each of the number of frequency ranges. This is beneficial since it allows for accurate compensation of noise when strong off-frequency sounds are perceived.

In a variant of the method, the audio speaker is comprised in an audio speaker assembly. Having the audio speaker in an assembly allows for a fixed position of the speakers and a more controlled and repeatable hearing test is possible. The noise perception model may further comprise applying a noise transfer model to the noise perception model. The noise transfer model comprises pre-calibrated data related to transfer of external noise into the audio speaker assembly. This is beneficial since it makes for a more accurate noise compensation since the actual noise transferred to the listener is compensated for.

In a second variant of the method, the method further comprises calculating test signal power adjustment levels for the one or more test tones based on the noise perception model and applying the signal power adjustment levels to the one or more test tones. By compensating the levels of the test signals, the result recorded will be accurate regardless of the environment, also, it is a simple and straight forward compensation that consumes little computing power.

In a third variant of the method, the method further comprises calculating test result adjustment levels for the number of test tone frequencies based on the noise perception model and applying the test result adjustment levels to the test result. By applying the signal to the test result, the result recorded will be accurate regardless of the environment. Also, it is a simple and straight forward compensation that consumes little computing power.

In a fourth variant of the method, evaluating the noise level further comprises, receiving a noise signal, dividing the noise signal into one or more frames, reducing false frequencies and transients in the one or more frames using windowing, over-lap and add techniques and transforming the one or more frames into a frequency domain using a Fast Fourier Transform. This is beneficial since the frequency representation offered by the FFT will be clean, stable and substantially free from unwanted artefacts.

According to the invention, the noise perception model is updated substantially continuously during the duration of the hearing test by substantially continuously evaluating the noise level. By running the method frequently, impulse noise and fast changes in noise are compensated for.

In a fifth variant of the method, determining the noise perception model further comprises applying a predetermined audio speaker calibration data comprising a frequency transfer function of the audio speaker. This is useful since it allows for the profile of the audio speakers to be unaffected by the compensation of the hearing test.

According to a second aspect, a method for compensating a hearing test is provided. The hearing test being performed by generating one or more test tones of a number of test tone frequencies, presenting, using an audio speaker, said test tones to a user, receiving user input indicative of the users perception of the test tones such that a test result comprising intensity levels of hearing for the number of test tone frequencies is provided, and generating a hearing profile based on the test result. The method comprises acquiring a pre-determined audio speaker calibration data comprising a frequency transfer function of the audio speaker, and compensating at least one of the test result or said one or more test tones using the pre-determined audio speaker calibration data.

According to a third aspect, an audio speaker assembly configured to be operatively connected to an interface unit of a computing device for performing a compensated hearing test according to the first aspect of the invention is provided. The computing device further comprises a first control unit and a first microphone. The audio speaker assembly comprises at least one audio speaker, a second microphone and a second control unit operatively connected to said at least one audio speaker. The second control unit is configured to control the at least one audio speaker to present one or more test tones of a number of test tone frequencies. The second control unit is further configured to, based on instructions received from the first control unit, perform at least one of, evaluate a noise level, perceived by the first and/or second microphone, determine a noise perception model based on said noise level, or compensate at least one of the test result or said one or more test tones using the noise perception model.

In a first variant of the audio speaker assembly the audio speaker assembly is further configured to perform the method of the second aspect.

According to a fourth aspect, a computing device configured to be operatively connected to an audio speaker assembly for performing a compensated hearing test according to the method of the first aspect is provided. The audio speaker assembly comprises at least one audio speaker, a second microphone and a second control unit. The computing device comprises an interface unit, a first microphone and a first control unit. The first control unit is configured to instruct the second control unit to, via the one or more audio speakers, present one or more test tones of a number of test tone frequencies. The first control unit is further configured to perform at least one of: evaluate a noise level, perceived by the first and/or second microphone, determine a noise perception model based on said noise level, or compensate at least one of the test result or said one or more test tones using the noise perception model.

In a first variant of the computing device, the computing device is further configured to perform the method of the second aspect.

According to a fifth aspect, a system for performing a compensated hearing test is provided. The hearing test being performed by allowing a user to indicate perception of one or more test tones of a number of test tone frequencies. The system comprising a computing device comprising an interface unit, a first microphone and a first control unit, an audio speaker assembly comprising at least one audio speaker, a second microphone and a second control unit. The first control unit is operatively connected to the second control unit and wherein the audio speaker assembly is the audio speaker assembly according to any of the third aspect or the computing device is the computing device according to any of the fourth aspect.

## BRIEF DESCRIPTION OF THE DRAWINGS

Embodiments of the invention will be described in the following; references being made to the appended diagrammatical drawings which illustrate non-limiting examples of how the inventive concept can be reduced into practice.

Fig. 1a is a system for performing a compensated hearing test in an uncontrolled environment according to one embodiment,
Fig. 1b is a block diagram for performing a compensated hearing test in an uncontrolled environment;
Fig. 2 is a block diagram of a noise evaluation module according to some embodiments;
Fig. 3 is a block diagram of a noise perception model determination module according to some embodiments;
Fig. 4a is a graph illustrating the concept of off-frequency masking,
Fig. 4b is a graph illustrating the concept of off-frequency masking;
Fig. 5 is a block diagram illustrating a method for noise compensating a hearing test performed in an uncontrolled environment according to some embodiments;
Fig. 6 is a detailed block diagram of the method step of evaluating a noise level according to some embodiments;
Fig. 7 is a detailed block diagram of the method step of determining a noise perception model according to some embodiments;
Fig. 8 is a block diagram illustrating a method for noise compensating a hearing test performed in an uncontrolled environment according to some embodiments;
Fig. 9 is a block diagram of an audio speaker assembly according to some embodiments;
Fig. 10 is a block diagram of a computing device according to some embodiments; and
Fig. 11 is a block diagram of a system for performing a compensated hearing test according to some embodiments.

## DETAILED DESCRIPTION OF EMBODIMENTS

Hereinafter, certain embodiments will be described more fully with reference to the accompanying drawings. The invention may, however, be embodied in many different forms and should not be construed as limited to the embodiments set forth herein; rather, these embodiments are provided by way of example so that this disclosure will be thorough and complete, and will fully convey the scope of the invention, such as it is defined in the appended claims, to those skilled in the art.

Sound pollution is an increasing problem and fewer and fewer places around the globe are considered silent. Any urban environment is subject to sounds from e.g. vehicles and transportation systems, environment control of buildings, neighbors etc. Some of these sounds exhibit some form of periodicity and repeatability in frequency behavior and intensity, others are completely stochastic, asynchronous and will occur once, such as particularly a car horn sounding in an intersection. Performing a hearing test in such an uncontrolled environment, as will be disclosed in this specification, has been made possible by inventive thinking of the inventors behind the solutions presented. A compensated hearing test, as will be disclosed in the coming sections, may be used to generate a hearing profile associated with a user or a listener such that the hearing profile may be used to compensate audio or audio playback equipment for any hearing impairments of the user. The hearing profile generated by some embodiments of this invention will make it possible to generate a generic hearing profile associated with one specific user. The hearing profile being generic in the sense that it describes the hearing profile of the user without any effects from the equipment used to generate the hearing profile. This enables the compensation and personalization of audio in any audio playback equipment even if the hearing test is done at a different location with different equipment. One non-limiting example where a generic hearing profile is beneficial is in an audio system for a vehicle, e.g. car, truck, bus, etc. It is challenging to generate the hearing profile in the vehicle, due to e.g. the difficulties associated with isolating the hearing of the left ear from the right ear.

Hearing impairment is, in this disclosure, meant as comprising any common deviation in perception of audio compared to a hearing profile of a person with perfect hearing. In other words, deafness or severe hearing disabilities is not considered a common deviation in perception of audio compared to a hearing profile of a person with perfect hearing.

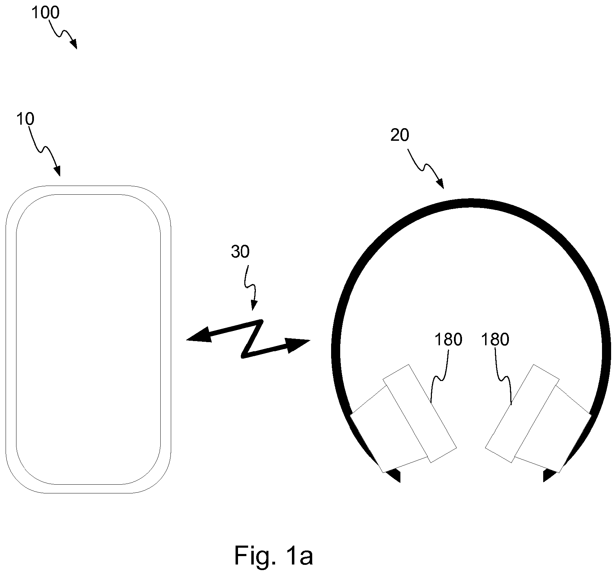

With reference to Fig. 1a, a system 100 for performing a compensated hearing test in an uncontrolled environment is shown. The system 100 comprises an audio speaker assembly 20, shown in Fig. 1a as a pair of headphones but it may very well be any suitable speaker assembly 20, as will be exemplified throughout this disclosure. Since the position of the listener in relation to the audio speaker 180 will affect the hearing profile, fixed position audio speaker assemblies 20 are preferred but this invention is not limited to such devices. Fixed position speaker assemblies 20 are here referred to as audio speaker assemblies 20 arranged in e.g. headphones or headsets in any size, shape or form.

The system 100 as illustrated in Fig. 1a, comprises a computing device 10, e.g. a mobile phone, PDA, laptop, smart watch or any suitable portable or stationary electronic equipment and an audio speaker assembly 20, e.g. a pair of headphones, a headset etc. The computing device 10 is operatively connected to the audio speaker assembly 20 via a communications interface 30. The communications interface 30 may be a wired or a wireless interface. The wireless interface may be any suitable interface e.g. Bluetooth, WIFI, cellular etc. The computing device 10 comprises an interface unit configured to be operatively connected to the audio speaker assembly 20 via the communications interface 30. The computing device 10 further comprises a first interface unit and a first microphone operatively connected to a first controller. The audio speaker assembly comprises at least one audio speaker 180 and a second control unit operatively connected to the audio speaker(s). The audio speaker assembly 20 may further comprise a second microphone.

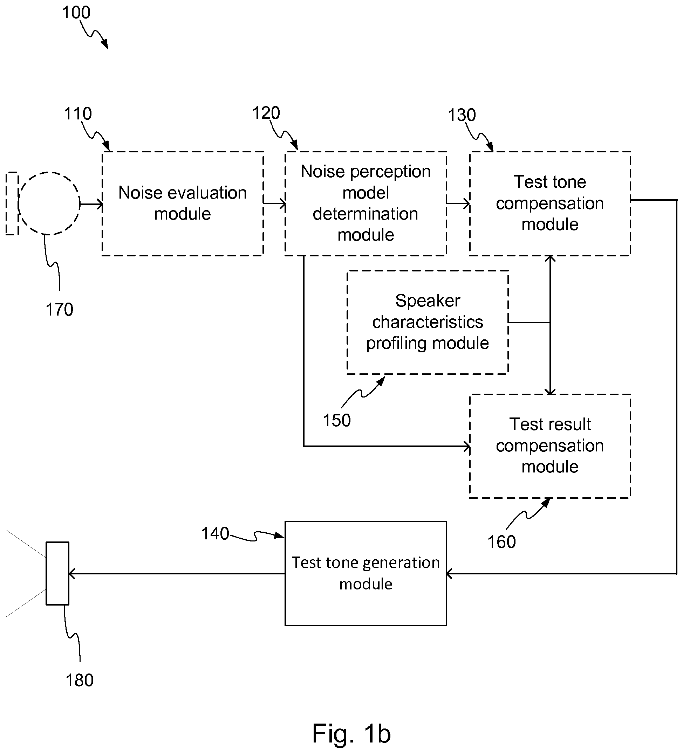

With reference to Fig. 1b, a block diagram of the system 100 for performing a compensated hearing test in an uncontrolled environment is shown. The system 100 comprises a microphone 170 that is operatively connected to a noise evaluation module 110 which in turn operatively connects to a noise perception model determination module 120. A test tone compensation module 130 receives its inputs from at least one of the noise perception model determination module 120 and a speaker characteristics profiling module 150. Alternatively, or additionally to the test tone compensation module 130 a test result compensation module 160 receives its input from at least one of the noise perception model determination module 120 and the speaker characteristics profiling unit 150. The test tone compensation module 130 is operatively connected to a test tone generation module 140 which operatively connects to an audio speaker 180. The audio speaker 180 is typically comprised in an audio speaker assembly 20. The modules 110, 120, 130, 140, 150, 160 of the system 100 will be explained in further detail in the coming section and it should be emphasized that not all modules 110, 120, 130, 140, 150, 160 are mandatory, even the microphone 170 may be omitted from some embodiments. It will be clear from the coming sections that the modules 110, 120, 130, 140, 150, 160 may be chosen and combined in many different combinations and all foreseeable combinations should be considered covered by this disclosure.

The following disclosure may detail the modules 110, 120, 130, 140, 150, 160 as standalone blocks. However, this is not necessarily the case and the modules 110, 120, 130, 140, 150, 160 should be considered as building blocks being realizable in many forms and combinable and dividable in any foreseeable manner. For instance, some or all of the modules 110, 120, 130, 140, 150, 160 may be hardware implemented and some or all may be software implemented. Further to this, some parts of a procedure relating to one particular module 110, 120, 130, 140, 150, 160 may be distributed between more than one unit, device or function arranged to perform subsets of that particular procedure.

Noise evaluation module

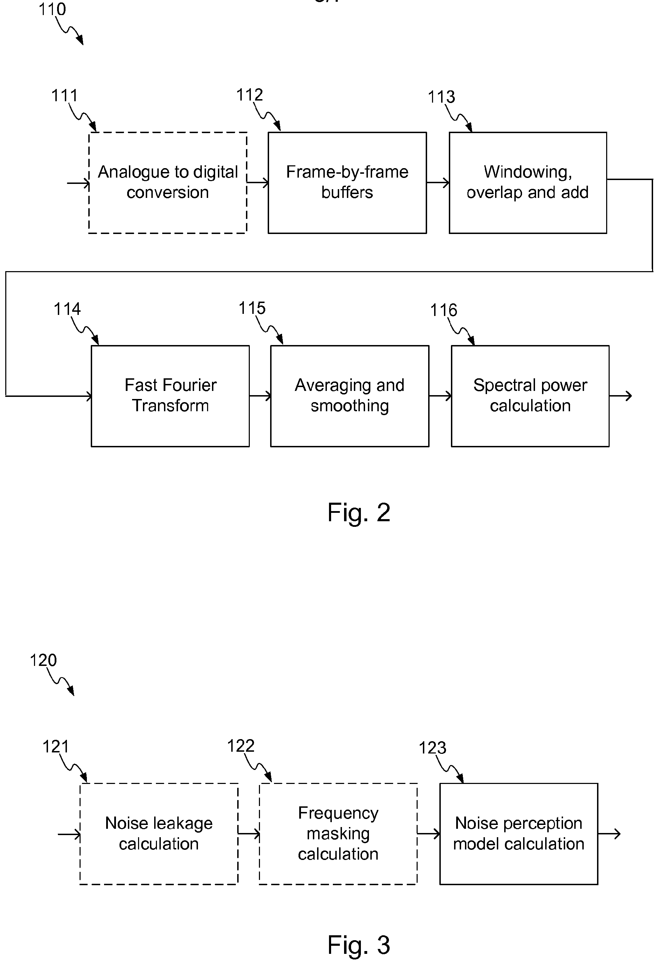

The noise evaluation module 110, detailed in Fig. 2, measures and evaluates a signal corresponding to a sound perceived by the microphone 170. The sound perceived by the microphone 170 may be provided to the noise evaluation module 110 in digital or analogue form. If the sound is provided in analogue form, the signal is sampled using e.g. an analogue to digital converter 111. The digital signal will have a sample rate Rs and each sample will be represented by a number of bits. The digital signal is divided into frames by buffering the samples in frame-by-frame buffers 112. The frames will contain N samples and the frame rate $R_F$ can consequently be calculated according to Eqn. 1.

$$R_F = \frac{R_S}{N} \qquad \text{Eqn. 1}$$

The frames may be of any length and the frame length affects the latency of the system. The latency will be manifested as how fast changes in sound can be tracked and changed. A typical frame length consists of 256 samples which corresponds to just under 6 ms of data in a 44100 Hz sampling audio system.

The frames are likely to comprise samples of signals of several different frequencies and the frequencies represented in a frame are unlikely an integer number of cycles of each frequency. These discontinuities in the frames give rise to spectral leakage when converted to a frequency domain and in order to reduce these effects, the frames are windowed. Windowing consists of multiplying samples of the frame by a window of the same length as the frame, where the amplitude of the widow varies smoothly and gradually towards zero at the edges of the window. The consequence is that the discontinuities, occurring at the edge of the frame, are reduced to zero which result in a frame that can be regarded as continuous. The windowing function reduces the energy of the frame and an overlap-and-add function is used in order to compensate for this loss of energy. The overlap-and-add function convolutes a current frame with a number of previous frames. The overlap-and-add functionality together with the windowing function is performed by the windowing, overlap-and-add function 113. There are numerous different windowing functions possible with e.g. varying roll-off rate and side lobe levels, typically in this disclosure, a Hamming or Hanning window may be suitable but these windowing functions should not be considered limiting. Also, the overlap-and-add function is configurable in e.g. the amount of overlap chosen, typically an overlap between 50 and 75% of the previous frame may be suitable but also this should be considered as a non-limiting example.

These frames are transformed into the frequency domain using e.g. a Fast Fourier Transform, FFT function 114 providing a frequency and phase representation of the audio. The frequency resolution of the frequency and phase representation of the audio will be dependent on both the frame length and the order of the FFT. In a non-limiting example, the FFT size is 256 which will result in a frequency resolution of half the FFT size, i.e. 128 frequency components or bins. Each bin will have a bandwidth of equal to the Nyquist sampling rate, i.e. half the sample rate of the audio signal, divided by the total number of bins. In the example with 44100 Hz sample rate and an FFT size of 256, the bandwidth of each bin will be 44100/2/128 which comes out as just above 170 Hz.

The frequency bins output from the FFT function 114 may vary and fluctuate between calculations. In order to reduce these fluctuations and find a more steady state of the signal, the signals are passed through an averaging and smoothing function 115. The averaging and smoothing may be performed by e.g. utilizing a sliding window function with or without weighting. A sliding window of a length N may be utilized to calculate the average across the current and the N-1 last samples. Alternatively or additionally, weighting may be applied where, typically, older samples arc multiplied with a lower factor compared to later samples. As a non-limiting example, consider two samples $S_n$ and $S_{n-1}$ being weighted by a weighting factor wf of 0.1, this would result in an average S according $\underline{S=S_n*(1-wf)+ S_{n-1}*wf}$. This averaging is in effect a low pass filter and will consequently reduce unwanted fluctuations and reduce possible false components of the signal. The skilled person will understand how to dimension averaging and smoothing parameters in order to achieve a suitable tradeoff between bandwidth and signal smoothness.

The comparably smooth signal being output from the averaging and smoothing function 115 is in turn fed to a spectral power calculation function 116. The spectral power calculation function 116 calculates the power spectrum of the signal for each bin by e.g. calculating the absolute value of the complex signal calculated by the FFT function 114, i.e. the magnitude of the signal.

Noise perception model determination module

The calculated power spectrum is fed to the noise perception model determination module 120, detailed in Fig. 3, which uses the received noise spectrum to calculate a noise perception model using a noise perception model calculation function 123. The noise perception model calculation function 123 calculates control values that may be used either to compensate the test tones using the test tone compensation module 130 and/or to compensate the test result using the test result compensation module 160. Basically, the control values calculated by the noise perception model calculation function 123 relates to an inverse of a perceived noise. The perceived noise may be the noise power spectrum as output from the spectral power calculation function 116. Alternatively, the perceived noise may be the noise power spectrum as output from the spectral power calculation function 116 modified by a noise leakage calculation function 121 and/or a frequency masking calculation function 122.

The received noise, as acquired by the microphone 170, is typically acquired at a position proximate to, but outside of, the audio speaker assembly 20. This means that the noise acquired by the microphone may be of a different level than the noise perceived by the listener due to e.g. noise attenuation of the audio speaker assembly 20. The noise leakage calculation function 121 compensates the noise power spectrum depending on how noise is transported from the outside of the audio speaker assembly 20, e.g. a pair of headphones, headset etc., to the ear of the listener. Basically this means describing how sounds of different frequencies are attenuated by the audio speaker assembly 20 and this may vary greatly depending on the audio speaker assembly 20. Many factors influence the noise leakage calculation e.g. the design of the audio speaker assembly 20 being e.g. an over-ear, on-ear or in-ear headset. The noise leakage of a particular speaker assembly is typically characterized once per unit, model, series, group of models, group of series or brand of audio speaker assemblies 20 and is stored in such a way that it is accessible and available to the noise evaluation module 121. The noise leakage may be characterized by utilizing two microphones. One microphone may be arranged in a position inside the audio speaker assembly 20 such that its perception of sound would be similar to that of the user of the audio speaker assembly 20. Another microphone may be arranged outside of the audio speaker assembly 20 and the noise leakage is characterized by the difference in sound pressure perceived by the two microphones for different frequencies. The noise leakage may be characterized for a number of different frequencies and also at different sound pressure levels. Since the noise leakage may be characterized at both different frequencies and at different sound pressures, the characterization may be represented in a noise leakage table. The noise leakage calculation function 121 module would, for each iteration depending on e.g. the power spectral density of a particular bin of the received noise spectrum, select appropriate data from the noise leakage table and compensate the received noise spectrum accordingly.

According to the invention, a function in the noise perception model determination module 120 is the frequency masking calculation function 122. The frequency masking calculation function 122 compensates the received noise spectrum for auditory masking occurring due to off-frequency masking. Simply put, this means that a sound at a first frequency may reduce the possibility to perceive sound at a second frequency, it can be compared to e.g. blocking and selectivity in radio electronics. The off-frequency masking is well described in literature with curves describing how sounds of different frequencies and intensity are masked by sounds at other frequencies and intensities. The effect of off-frequency masking is used in the compression algorithms for audio e.g. the Moving Picture Experts Group Layer-3 Audio, MP3. In order for off-frequency masking to occur, the off-frequency signal has to have a higher sound pressure level than the wanted sound. This means that the frequency masking calculation function 122 may be applied adaptively depending on the received noise spectrum and also on the expected sound pressure of the test tone to be generated. In other words, the frequency masking calculation function 122 is only needed if there are frequency bins surrounding a frequency bin currently targeted for the hearing test that have higher sound pressure level than the frequency bin of interest.

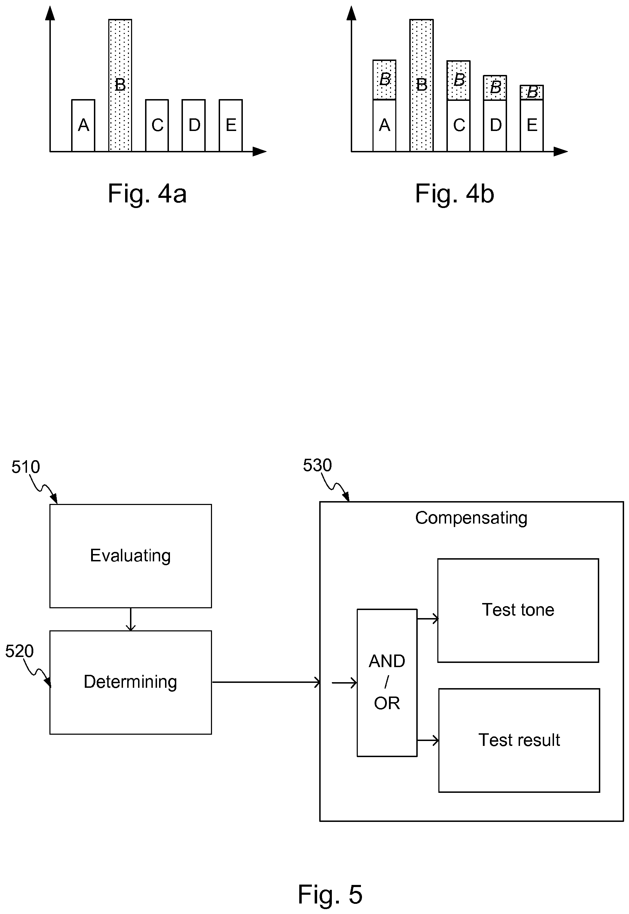

A non-limiting example is here presented with reference to Figs. 4a and 4b. In Fig. 4a, a received noise spectrum represented in five bins A-E is illustrated. Bin B has a comparably higher power spectral density compared to the other bins A, C, D, E and bin B may mask the other bins A, C, D, E. For completeness, the form of auditory masking the received noise power in bin B will have on frequencies falling within the bandwidth of bin B is referred to as on-frequency masking. The off-frequency masking effect bin B has on the other bins A, C, D, E is shown in Fig. 4b where the masking effect is illustrated as an addition in the power spectral density of the other bins A, C, D, E.

The noise perception model calculation function 123 uses the received noise power, optionally subject to the noise leakage calculation function 121 and/or the frequency masking calculation function to generate a noise perception model. The noise perception model comprises, for each bin, a compensation level indicative of how a test tone or test result, falling within a corresponding bin should be compensated. Typically, a test tone or its corresponding test result is increased by the received noise power, optionally subject to the noise leakage calculation function 121 and/or the frequency masking calculation function 122 multiplied with a scaling factor. The scaling factor may be determined empirically and may be the same factor for all of the bins or some of the bins. The noise perception model is provided as an input to the test tone compensation module 130 and/or the test result compensation module 160.

Speaker characteristics profiling module

The speaker characteristics profiling module 150 is an optional module, function or unit that calculates the effect of an audio speaker assembly 20. Many manufacturers of audio speaker assemblies 20 and audio speakers 180 have models with characteristic sounds or have a particular sound that they would like to associate with their brand. Some audio speaker assemblies 20 may be designed with characteristics specific to particular styles of music such as rock. An audio speaker assembly 20 with characteristics aimed at providing a rock-like sound may have a frequency response that is flat in the middle regions and amplified or increased in the lower and higher regions. When performing a hearing test in order to compensate sound for the hearing profile of the listener, the effect of the audio speaker assembly 20 used when performing the hearing test to generate the hearing profile will be compensated for. The hearing test will aim at generating a substantially flat which means that the frequency response associated with an audio speaker assembly 20 used when performing a hearing test will be compensated for by the hearing test. This means that designed characteristics of an audio speaker assembly 20 will be removed and this may not be a desired effect. The speaker characteristics profiling module 150 has access to a headset calibration data. The headset calibration data is provided pre-calculated and pre-stored in such a manner that it is accessible by the speaker characteristics profiling module 150. The headset calibration data may be e.g. stored in a local memory, accessible from a cloud service, provided by a user etc. The headset calibration data is typically generated by feeding an audio speaker assembly 20 with white noise or several different frequencies one by one and measuring the auditory response from the audio speaker assembly 20. The headset calibration data may be characterized by the difference between the sound pressure level of the sound fed to the audio speaker 180 and the sound pressure level as measured as the auditory response from the audio speaker 180. The speaker characteristics profiling module 150 uses the headset calibration data to generate a speaker characteristic profile which may be the inverse of the headset calibration data that may further be scaled by a factor. Typically, if the headset calibration data indicate a reduction of sound pressure of a particular magnitude at a particular frequency, the speaker characteristic profile comprises an increase of substantially the same magnitude at substantially the same frequency. The speaker characteristic profile is provided as an optional input to the test tone compensation module 130 and/or the test result compensation module 160.

## Test tone compensation module

The test tone compensation module 130 receives input from at least one of the noise perception model determination module 120 or from the speaker characteristics profiling module 150 and uses these inputs to form a test tone compensation that is provided to the test tone generation module 140. The test tone compensation may comprise e.g. factors or compensation values each associated directly with the different frequencies of the test tones. It may be that the output of the speaker characteristics profiling module 150 is in a different format to the output from the noise perception model determination module 120 and/or the desired input of the test tone generation module 140. The test tone compensation module 130 converts, if necessary, its inputs to the desired format of the test tone generation module. The resolution in frequency or amplitude may differ between either the input and/or the output and the test tone compensation module 130 scales the inputs to correspond to the desired input of the test tone generation module 140.

## Test result compensation module

The test result compensation module 160 is an alternative or addition to the test tone compensation module 130. The test result compensation module 160 receives inputs from at least one of the noise perception model determination module 120 or the speaker characteristics profiling module 150. These inputs are used to compensate the result of the hearing test and that may be done e.g. continuously as the test progresses, at the end of the test or at the certain steps of the hearing test such as upon finalization of one particular test tone.

## Test tone generation module

The test tone generation module 140 is the unit, module, device or function responsible for generating the test tones that are provided to the audio speaker 180. The test tone generation module 140 receives input, the test tone compensation, from the test tone compensation module 130 and applies the test tone compensation to the test tone to be generated. Typically this may imply increasing the sound pressure of a test tone in the presence of ambient noise and perhaps decreasing a test tone to compensate for an amplification of an audio speaker assembly 20. In either way, the test tone compensation comprises the necessary data to perform the compensation of the test tone in order to provide a test tone of desired amplitude and frequency to the speaker assembly 180.

As is apparent from the disclosure so far, the combination of the functions, modules overviewed in Fig. 1 may be done in many different ways. The following sections will detail some of these combinations, but it should be mentioned that all suitable combinations are to be considered covered although not explicitly disclosed.

A preferred embodiment of a method for noise compensating a hearing test performed in an uncontrolled environment is presented in Fig. 5. The method may be performed by e.g. the system 100 presented in Fig. 1b. The hearing test is performed by generating, by e.g. a test tone generation module 140, one or more test tones and providing the one or more test tones to e.g. an audio speaker 180, so that they can be presented to a user. The user indicates perception of the audio tones by providing feedback that is communicated to the test tone generation module 140 so that the intensity of the test tone associated with the feedback can be adjusted in order to find the hearing threshold of the user at that frequency. The result of the hearing test will comprise a number of intensity levels of hearing, typically one per test tone frequency, and a hearing profile can be generated from the test result. The method comprises evaluating 510 a noise level, which may be done by the previously presented noise level evaluation module 110 receiving noise from a microphone 170. Determining 520, by e.g. the noise perception model calculation module 123 known from previous section, a noise perception model based on the evaluated noise level. The method further comprises compensating 530, based on the noise perception model, either or both of the test result and the one or more test tones. Compensating 530 may be done by e.g. the previously presented test tone compensation module 130 and/or the previously presented test result compensation module 160.

The step of evaluating 510 may in some variants of the embodiment comprise the step of calculating 660 a noise power spectrum. This is performed by a suitable function, e.g. the presented spectral power calculation function 116.

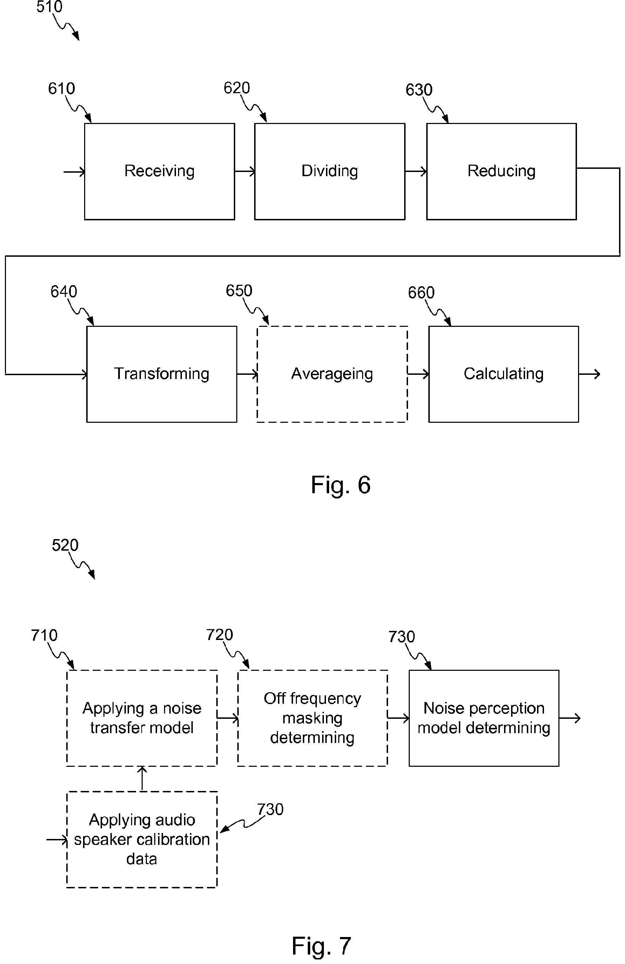

In further variants, the step of evaluating 510 comprises the steps detailed in Fig. 6. Evaluating 510 is initiated by the reception 610 of a noise signal, this step is performed by the previously presented analogue to digital conversion module 111 in combination with e.g. the microphone 170. The received signal is subjected to the step of dividing 620 the signal into one or more frames which is performed by the frame-by-frame buffers 112 as presented in earlier sections. The framed signal is processed by reducing 630 false frequencies and transients using windowing, over-lap and add techniques. Typically, this is performed by a function such as the disclosed windowing, overlap and add function 113. The reduced signal is further processed by transforming 640 the frames into the frequency domain using a Fast Fourier Transform by functions, processes and/or devices as disclosed with relation to the Fast Fourier Transform block 114. Optionally, the step of evaluating 510 may comprise the step of averaging 650 the transformed signal. This may be done according to functions presented in relation to the averaging and smoothing function 115.

In some variants of the method, the step of determining 520 is performed as detailed in Fig. 7. The noise perception model determining 730 is, as mentioned above, performed by functions, modules or blocks implemented in accordance with the noise perception model calculation 123 as presented in earlier sections. According to the invention, the step of determining 520 also comprises the step of off frequency masking determining 720 which is performed in accordance with the details given regarding the frequency masking calculation 122. Further to this variant, or as a separate variant, the step of determining 520 may comprise the step of applying a noise transfer model 710 which is conducted in line with the description given regarding the noise leakage calculation 121 in previous sections. The step of determining 520 may comprise, in some variants, the step of applying 730 a pre-determined audio speaker calibration data. This is preferably done in line with the description given regarding the speaker characteristics profiling module 150 as earlier presented.

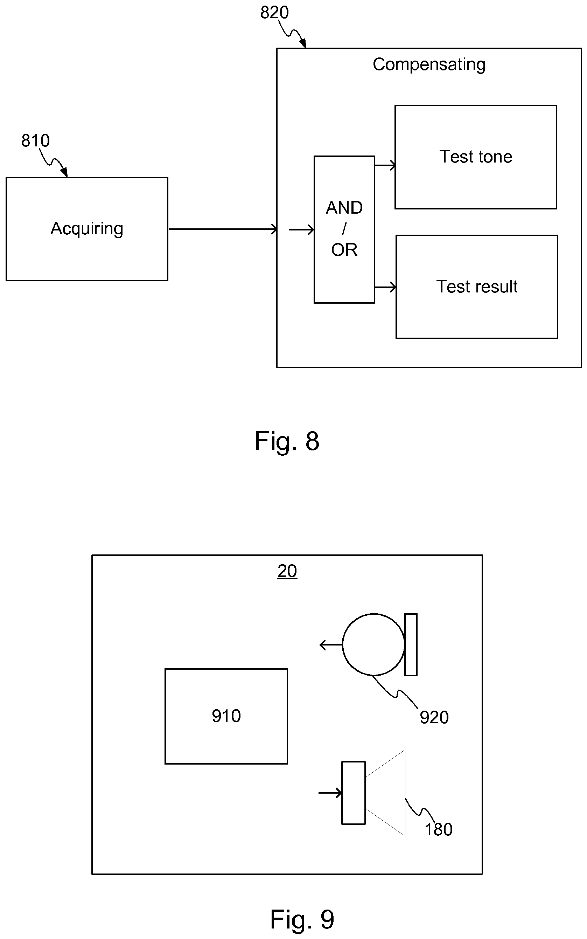

In another preferred embodiment, presented in Fig. 8, a method for compensating a hearing test is disclosed. The hearing test is performed by generating one or more test tones of a number of test tone frequencies. The test tones arc presented to the user using e.g. an audio speaker 180. The user will indicate perception of the test tone such that a test result comprising intensity levels of hearing for the number of test tone frequencies is provided. Based on the test result a hearing profile is created by the hearing test. The method comprises the step of acquiring 810 audio speaker calibration data. This data is typically predetermined and more details regarding the speaker calibration data and the compensation are to be found in the section relating to the speaker characteristics profiling module 150. Compensating 820 may be done by e.g. the previously presented test tone compensation module 130 and/or the previously presented test result compensation module 160.

In Fig. 9, a block diagram of a preferred embodiment of an audio speaker assembly 20 is presented. The audio speaker assembly 20 is configured to connect to an interface unit 1030 of a computing device 10 such that a compensated hearing test as described in earlier passages of this disclosure is possible to perform. The audio speaker assembly 20 comprises a second controller 910, the at least one audio speaker 180 and a second microphone 920. The second control unit 910 is operatively connected to the audio speaker 180 and, in some variants, also to the second microphone 920. The connection to the interface unit 1030 may be any suitable connection interface and the skilled person will have the knowledge to adapt the disclosed audio speaker assembly 20 such that a suitable operative connection is achieved between the audio speaker assembly 20 and the interface unit 1030 of the computing device 10. The second control unit 910 receives instructions from the interface unit 1030 of the computing device 10 and these instructions comprise instruction to do either some of or all of evaluate a noise level, determine a noise perception model based on a noise level and/or compensate at least one of the test result or the test tones using a noise perception model. The data necessary to execute the steps, if the second controller 910 is not instructed to obtain, by e.g. calculation, evaluation, measurement etc., the data, will be received from the interface unit 1030. Such data is e.g. raw noise data, evaluated noise level, noise perception model etc. It may be that the data obtained by the second control unit 910 is to be sent back to the interface unit 1030 of the computing device 10 for e.g. further processing, storage, validation etc. The execution of the instructions, evaluating a noise level, determining a noise perception model or compensating at least one of the test result or the test tones is performed as outlined in previous sections of this disclosure.

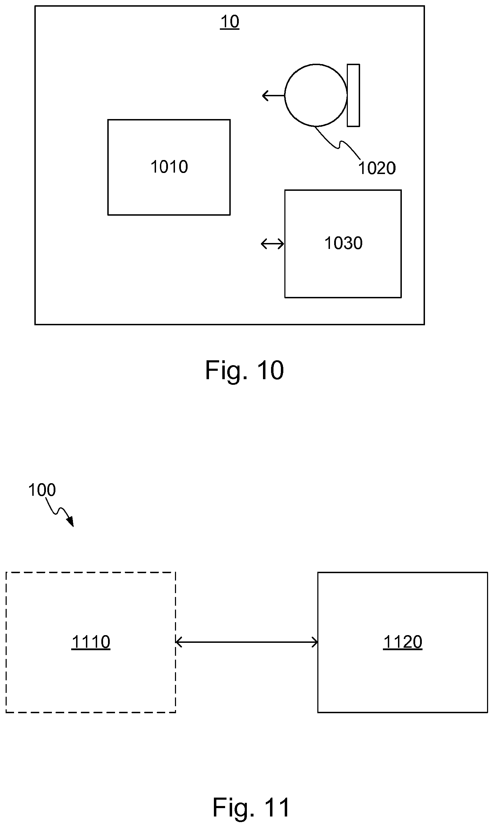

With reference to Fig. 10, which illustrates a block diagram of the computing device 10, a preferred embodiment of the computing device 10 will be presented. The computing device 10 is configured, or at least configurable, to be operatively connected to the audio speaker assembly 20 such that a compensated hearing test as presented in earlier sections is possible to perform. The computing device 10 comprises the first controller 1010, a first microphone 1020 and the interface unit 1030. The first control unit 1010 is configured, or configurable, to operatively connect, via the interface unit 1030, to the second control unit 910 of the audio speaker assembly 20. The first control unit 1010 will send instructions to the second control unit 910 comprising instruction to do at least one of presenting one or more test tones of a number of test tone frequencies, evaluate a noise level, determine a noise perception model and/or compensate at least one of the test result or one or more test tones. The instructions sent to the second control unit 910 may in some variants comprise the necessary data to execute the requested instructions, examples of data is e.g. raw noise data, evaluated noise level, noise perception model etc.

Fig. 11 presents a block diagram of one embodiment of the system 100 for performing a compensated hearing test according to this disclosure. The system 100 comprises a computing device 1110 and an audio speaker assembly 1120. The system 100 further comprises at least one control unit, at least one microphone and an audio speaker 180. The at least one microphone is used to perceive noise that is sent to the at least one control unit. The at least one audio speaker 180 is used for presenting the previously described one or more test tones of a number of test tone frequencies to a user. The functions outlined in e.g. Fig. 1b and further detailed with reference to the following Figs. are all possible to be performed by the system 100.

In one variant, the computing device 1010 is the only device in the system comprising a control unit and this control unit would be responsible for performing all the functions disclosed. Such a system is exemplified with the computing device 1010 being e.g. a mobile phone and the audio speaker assembly 1020 is e.g. a pair of wired headphones.

In another variant, the audio speaker assembly also comprise a control unit but all functions relating to the compensated hearing test are being performed by the computing device 1110. An example is a system 100 wherein the computing device 1010 is e.g. a mobile phone and the audio speaker assembly 1020 is e.g. a pair of active wired or wireless headphones such as noise cancelling headphones etc.

In another variant, the computing device 1110 is optional and the audio speaker assembly 1020 is solely responsible for performing the compensated hearing test. This is exemplified with the audio speaker assembly 1120 being a pair of intelligent headphones that can generate test tones and receive user feedback, through e.g. buttons or other means of control, indicative of user perception of the test tones.

From the presentation above of the two extreme variants of the system 100, one where the complete compensated hearing test is performed by the computing device 1110 and the other where the complete compensated hearing test is performed by the audio speaker assembly 1120, it is clear that either device 1110, 1120 may be configured to perform either part of the compensated hearing test. No particular function, except the presentation of the test tones to the user, is tied to one particular device 1110, 1120. Also, the functions and methods as disclosed may be divided between the devices 1110, 1120 of the system 100.

However, in a preferred embodiment of the system 100 the computing device 1110 of Fig. 11 is the computing device 10 as presented in previous sections and the audio speaker assembly 1120 of Fig. 11 is the audio speaker assembly 20 of previous sections.

## Claims

1. A method for noise compensating a hearing test performed in an uncontrolled environment, the hearing test being performed by generating one or more test tones of a number of test tone frequencies, presenting, using an audio speaker (180), said test tones to a user, receiving user input indicative of the users perception of the test tones such that a test result comprising intensity levels of hearing for the number of test tone frequencies is provided, and generating a hearing profile based on the test result, wherein the method comprises:

evaluating (510) a noise level received from a microphone (170) and calculating (660) a noise power spectrum based on the evaluated noise level, the noise power spectrum comprising noise level for a number of frequency ranges, determining (520) a noise perception model based on said noise level and off frequency masking (122) between the one or more test tone frequencies and the noise power spectrum for each of the number of frequency ranges, wherein the noise perception model is updated substantially continuously during the duration of the hearing test by substantially continuously evaluating the noise level and compensating (530) the test result and said one or more test tones using the noise perception model.

2. The method according to claim 1, wherein the number of frequency ranges is the same as the number of test tone frequencies.

3. The method according any of the preceding claims, wherein the audio speaker (180) is comprised in an audio speaker assembly (20).

4. The method according to claim 3, wherein the noise perception model further comprises applying a noise transfer model (710) to the noise perception model, wherein the noise transfer model comprises pre-calibrated data related to transfer of external noise into the audio speaker assembly (20).

5. The method according to any of the preceding claims, wherein the method further comprises calculating test signal power adjustment levels for the one or more test tones based on the noise perception model and applying the signal power adjustment levels to the one or more test tones.

6. The method according to any of the preceding claims, wherein the method further comprises calculating test result adjustment levels for the number of test tone frequencies based on the noise perception model and applying the test result adjustment levels to the test result.

7. The method according any of the preceding claims, wherein evaluating (510) the noise level further comprises,

receiving (610) a noise signal, dividing (620) the noise signal into one or more frames, reducing (630) false frequencies and transients in the one or more frames using windowing, over-lap and add techniques,
transforming (640) the one or more frames into a frequency domain using a Fast Fourier Transform.

8. The method according to any of the preceding claims, wherein determining (520) the noise perception model further comprises applying (730) a pre-determined audio speaker calibration data comprising a frequency transfer function of the audio speaker (180).

9. An audio speaker assembly (20) configured to be operatively connected to an interface unit (1030) of a computing device (10) for performing a compensated hearing test according to the method of any of the claims 1 to 8, wherein the computing device (10) further comprises a first control unit (1010) and a first microphone (1020), wherein the audio speaker assembly (20) comprises at least one audio speaker (180), a second microphone (920) and a second control unit (910) operatively connected to said at least one audio speaker (180), wherein the second control unit (910) is configured to control the at least one audio speaker (180) to present one or more test tones of a number of test tone frequencies, and wherein the second control unit (910) is further configure to, based on instructions received from the first control unit (1010), perform:

   substantially continuously during the duration of the hearing test, evaluate a noise level, perceived by the first and/or second microphone (920, 1020) and calculate a noise power spectrum based on the evaluated noise level, the noise power spectrum comprising noise level for a number of frequency ranges,
   determine a noise perception model based on said noise level and off frequency masking (122) between the one or more test tone frequencies and the noise power spectrum for each of the number of frequency ranges, wherein the noise perception model is updated substantially continuously during the duration of the hearing test by substantially continuously evaluating the noise level, or
   compensate the test result and said one or more test tones using the noise perception model.

10. The audio speaker assembly (20) according to claim 9, wherein the second control unit (910) is further configure to, based on instructions received from the first control unit (1010):

    acquire a pre-determined audio speaker calibration data comprising a frequency transfer function of the audio speaker (180),
    compensate (820) at least one of the test result or said one or more test tones using the pre-determined audio speaker calibration data.

11. A computing device (10) configured to be operatively connected to an audio speaker assembly (20) for performing a compensated hearing test according to the method of any of the claims 1 to 8, the audio speaker assembly (20) comprising at least one audio speaker (180), a second microphone (920) and a second control unit (910), wherein the computing device (10) comprises:

    an interface unit (1030), a first microphone (1020) and a first control unit (1010);
    wherein the first control unit (1010) is configured to instruct the second control unit (910) to, via the one or more audio speakers (180), present one or more test tones of a number of test tone frequencies, and
    wherein the first control unit (1010) is further configured to perform:

       substantially continuously during the duration of the hearing test, evaluate a noise level, perceived by the first and/or second microphone (920, 1020),
       determine a noise perception model based on said noise level and off frequency masking (122) between the one or more test tone frequencies and the noise power spectrum for each of the number of frequency ranges, wherein the noise perception model is updated substantially continuously during the duration of the hearing test by substantially continuously evaluating the noise level, or compensate the test result and said one or more test tones using the noise perception model.

12. The computing device (10) according to claim 11, wherein the computing device (10) is further configured to:

    acquire a pre-determined audio speaker calibration data comprising a frequency transfer function of the audio speaker (180),
    compensate (820) at least one of the test result or said one or more test tones using the pre-determined audio speaker calibration data.

13. A system (100) for performing a compensated hearing test, the hearing test being performed by allowing a user to indicate perception of one or more test tones of a number of test tone frequencies, the system (100) comprising:

    a computing device (10) comprising an interface unit (1030), a first microphone (1020) and a first control unit (1010),
an audio speaker assembly (20) comprising at least one audio speaker (180), a second microphone (920) and a second control unit (910), wherein
the first control unit (1010) is operatively connected to the second control unit (910) and wherein the audio speaker assembly (20) is the audio speaker assembly (20) according to any of the claims 9 or 10 or the computing device (10) is the computing device (10) according to any of the claims 11 or 12.

**Patentansprüche**

1. Verfahren zur Geräuschkompensation eines Hörtests, der in einer unkontrollierten Umgebung erfolgt, wobei der Hörtest erfolgt, indem ein oder mehrere Testtöne einer Anzahl von Testtonfrequenzen erzeugt werden, die Testtöne unter Verwendung eines Audiolautsprechers (180) einem Nutzer präsentiert werden, eine Nutzereingabe empfangen wird, die eine Wahrnehmung der Testtöne durch den Nutzer angibt, sodass ein Testergebnis, das Intensitätspegel des Hörens für die Anzahl von Testtonfrequenzen umfasst, bereitgestellt wird, und ein Hörprofil basierend auf dem Testergebnis erzeugt wird, wobei das Verfahren umfasst

   Auswerten (510) eines von einem Mikrofon (170) empfangenen Geräuschpegels und Berechnen (660) eines Geräuschleistungsspektrums basierend auf dem ausgewerteten Geräuschpegel, wobei das Geräuschleistungsspektrum Geräuschpegel für eine Anzahl von Frequenzbereichen umfasst,
   Bestimmen (520) eines Geräuschwahrnehmungsmodells basierend auf dem Geräuschpegel und einer Außerfrequenz-Maskierung (122) zwischen der einen oder den mehreren Testtonfrequenzen und dem Geräuschleistungsspektrum für jeden der Anzahl von Frequenzbereichen, wobei das Geräuschwahrnehmungsmodell während der Dauer des Hörtests im Wesentlichen kontinuierlich aktualisiert wird, indem der Geräuschpegel im Wesentlichen kontinuierlich ausgewertet wird, und
   Kompensieren (530) des Testergebnisses und des einen oder der mehreren Testtöne unter Verwendung des Geräuschwahrnehmungsmodells.

2. Verfahren nach Anspruch 1, wobei die Anzahl von Frequenzbereichen dieselbe ist wie die Anzahl von Testtonfrequenzen.

3. Verfahren nach einem der vorhergehenden Ansprüche, wobei der Audiolautsprecher (180) in einer Audiolautsprecheranordnung (20) enthalten ist.

4. Verfahren nach Anspruch 3, wobei das Geräuschwahrnehmungsmodell ferner Anwenden eines Geräuschübertragungsmodells (710) auf das Geräuschwahrnehmungsmodell umfasst, wobei das Geräuschübertragungsmodell vorkalibrierte Daten in Bezug auf die Übertragung von Außengeräusch in die Audiolautsprecheranordnung (20) umfasst.

5. Verfahren nach einem der vorhergehenden Ansprüche, wobei das Verfahren ferner Berechnen von Testsignalleistungsanpassungspegeln für den einen oder die mehreren Testtöne basierend auf dem Geräuschwahrnehmungsmodell und Anwenden der Signalleistungsanpassungspegel auf den einen oder die mehreren Testtöne umfasst.

6. Verfahren nach einem der vorhergehenden Ansprüche, wobei das Verfahren ferner Berechnen von Testergebnisanpassungspegeln für die Anzahl von Testtonfrequenzen basierend auf dem Geräuschwahrnehmungsmodell und Anwenden der Testergebnisanpassungspegel auf das Testergebnis umfasst.

7. Verfahren nach einem der vorhergehenden Ansprüche, wobei Auswerten (510) des Geräuschpegels ferner umfasst

   Empfangen (610) eines Geräuschsignals,
   Unterteilen (620) des Geräuschsignals in einen oder mehrere Rahmen,
   Reduzieren (630) falscher Frequenzen und Transienten in dem einen oder den mehreren Rahmen unter Verwendung von Windowing-, Überschneidungs- und Additionstechniken,
   Transformieren (640) des einen oder der mehreren Rahmen in einen Frequenzbereich unter Verwendung einer schnellen Fourier-Transformation.

8. Verfahren nach einem der vorhergehenden Ansprüche, wobei Bestimmen (520) des Geräuschwahrnehmungsmodells ferner Anwenden (730) vorbestimmter Audiolautsprecherkalibrierungsdaten umfasst, die eine Frequenzübertragungsfunktion des Audiolautsprechers (180) umfassen.

9. Audiolautsprecheranordnung (20), die konfiguriert ist, mit einer Schnittstelleneinheit (1030) einer Rechenvorrichtung (10) betriebswirksam verbunden zu sein, um einen kompensierten Hörtest gemäß dem Verfahren nach einem der Ansprüche 1 bis 8 durchzuführen, wobei die Rechenvorrichtung (10) ferner eine erste Steuereinheit (1010) und ein erstes Mikrofon (1020) aufweist, wobei die Audiolautsprecheranordnung (20) mindestens einen Audiolautsprecher (180), ein zweites Mikrofon (920) und eine zweite Steuereinheit (910) aufweist, die mit dem mindestens einen Audiolautsprecher (180) betriebswirksam verbunden ist, wobei die zweite Steuereinheit (910) konfiguriert ist, den mindestens einen Audiolautsprecher (180) zu steuern, um einen oder mehrere Testtöne einer Anzahl von Testtonfrequenzen zu präsentieren, und

wobei die zweite Steuereinheit (910) ferner konfiguriert ist, basierend auf Anweisungen, die von der ersten Steuereinheit (1010) empfangen werden, durchzuführen

im Wesentlichen kontinuierlich während der Dauer des Hörtests einen Geräuschpegel auszuwerten, der durch das erste und/oder zweite Mikrofon (920, 1020) wahrgenommen wird, und ein Geräuschleistungsspektrum basierend auf dem ausgewerteten Geräuschpegel zu berechnen, wobei das Geräuschleistungsspektrum Geräuschpegel für eine Anzahl von Frequenzbereichen umfasst,

ein Geräuschwahrnehmungsmodell basierend auf dem Geräuschpegel und einer Außerfrequenz-Maskierung (122) zwischen der einen oder den mehreren Testtonfrequenzen und dem Geräuschleistungsspektrum für jeden der Anzahl von Frequenzbereichen zu bestimmen, wobei das Geräuschwahrnehmungsmodell während der Dauer des Hörtests im Wesentlichen kontinuierlich aktualisiert wird, indem der Geräuschpegel im Wesentlichen kontinuierlich ausgewertet wird, oder

das Testergebnis und den einen oder die mehreren Testtöne unter Verwendung des Geräuschwahrnehmungsmodells zu kompensieren.

10. Audiolautsprecheranordnung (20) nach Anspruch 9, wobei die zweite Steuereinheit (910) ferner konfiguriert ist, basierend auf von der ersten Steuereinheit (1010) empfangenen Anweisungen:

vorbestimmte Audiolautsprecher-Kalibrierungsdaten zu erfassen, die eine Frequenzübertragungsfunktion des Audiolautsprechers (180) umfassen,

das Testergebnis und/oder den einen oder die mehreren Testtöne unter Verwendung der vorbestimmten Audiolautsprecher-Kalibrierungsdaten zu kompensieren (820).

11. Rechenvorrichtung (10), die konfiguriert ist, mit einer Audiolautsprecheranordnung (20) betriebswirksam verbunden zu sein, um einen kompensierten Hörtest gemäß dem Verfahren nach einem der Ansprüche 1 bis 8 durchzuführen, wobei die Audiolautsprecheranordnung (20) mindestens einen Audiolautsprecher (180), ein zweites Mikrofon (920) und eine zweite Steuereinheit (910) aufweist, wobei die Rechenvorrichtung (10) aufweist:

eine Schnittstelleneinheit (1030), ein erstes Mikrofon (1020) und eine erste Steuereinheit (1010);

wobei die erste Steuereinheit (1010) konfiguriert ist, die zweite Steuereinheit (910) anzuweisen, über den einen oder die mehreren Audiolautsprecher (180) einen oder mehrere Testtöne einer Anzahl von Testtonfrequenzen zu präsentieren, und

wobei die erste Steuereinheit (1010) ferner konfiguriert ist, durchzuführen:

während der Dauer des Hörtests im Wesentlichen kontinuierlich einen Geräuschpegel auszuwerten, der von dem ersten und/oder zweiten Mikrofon (920, 1020) wahrgenommen wird,

ein Geräuschwahrnehmungsmodell basierend auf dem Geräuschpegel und einer Außerfrequenz-Maskierung (122) zwischen der einen oder den mehreren Testtonfrequenzen und dem Geräuschleistungsspektrum für jeden der Anzahl von Frequenzbereichen zu bestimmen, wobei das Geräuschwahrnehmungsmodell während der Dauer des Hörtests im Wesentlichen kontinuierlich aktualisiert wird, indem der Geräuschpegel im Wesentlichen kontinuierlich ausgewertet wird, oder

das Testergebnis und den einen oder die mehreren Testtöne unter Verwendung des Geräuschwahrnehmungsmodells zu kompensieren.

12. Rechenvorrichtung (10) nach Anspruch 11, wobei die Rechenvorrichtung (10) ferner konfiguriert ist:

vorbestimmte Audiolautsprecher-Kalibrierungsdaten zu erfassen, die eine Frequenzübertragungsfunktion des Audiolautsprechers (180) umfassen,

das Testergebnis und/oder den einen oder die mehreren Testtöne unter Verwendung der vorbestimmten Audiolautsprecher-Kalibrierungsdaten zu kompensieren (820).

13. System (100) zum Durchführen eines kompensierten Hörtests, wobei der Hörtest erfolgt, indem es einem Nutzer ermöglicht wird, eine Wahrnehmung eines oder mehrerer Testtöne einer Anzahl von Testtonfrequenzen anzugeben, wobei das System (100) aufweist:

eine Rechenvorrichtung (10), die eine Schnittstelleneinheit (1030), ein erstes Mikrofon (1020) und eine erste Steuereinheit (1010) aufweist, eine Audiolautsprecheranordnung (20), die mindestens einen Audiolautsprecher (180), ein zweites Mikrofon (920) und eine zweite Steuereinheit (910) aufweist, wobei

die erste Steuereinheit (1010) mit der zweiten Steuereinheit (910) betriebswirksam verbunden ist und wobei die Audiolautsprecheranordnung (20) die Audiolautsprecheranordnung (20) nach einem der Ansprüche 9 oder 10 ist oder die Rechenvorrichtung (10) die Rechenvorrichtung (10) nach einem der Ansprüche 11 oder 12 ist.

## Revendications

1. Procédé de compensation du bruit d'un test auditif réalisé dans un environnement non contrôlé, le test auditif étant réalisé par génération d'une ou plusieurs tonalités de test d'un nombre de fréquences de tonalités de test, par présentation, à l'aide d'un haut-parleur audio (180), desdites tonalités de test à un utilisateur, par réception d'une entrée utilisateur indiquant la perception par l'utilisateur des tonalités de test de telle sorte qu'un résultat de test comprenant des niveaux d'intensité d'audition pour le nombre de fréquences de tonalités de test est fourni, et par génération d'un profil d'audition sur la base du résultat du test, dans lequel le procédé comprend :

   l'évaluation (510) d'un niveau de bruit reçu d'un microphone (170) et le calcul (660) d'un spectre de puissance du bruit sur la base du niveau de bruit évalué, le spectre de puissance du bruit comprenant un niveau de bruit pour un nombre de plages de fréquence,
   la détermination (520) d'un modèle de perception du bruit sur la base dudit niveau de bruit et d'un masquage hors fréquence (122) entre les une ou plusieurs fréquences de tonalités de test et le spectre de puissance du bruit pour chacune du nombre de plages de fréquence, dans lequel le modèle de perception du bruit est mis à jour de manière sensiblement continue pendant la durée du test auditif en évaluant de manière sensiblement continue le niveau de bruit et
   la compensation (530) du résultat de tests et desdites une ou plusieurs tonalités de test en utilisant le modèle de perception du bruit.

2. Procédé selon la revendication 1, dans lequel le nombre de plages de fréquence est le même que le nombre de fréquences de tonalités de test.

3. Procédé selon l'une quelconque des revendications précédentes, dans lequel le haut-parleur audio (180) est composé d'un ensemble (20) haut-parleur audio.

4. Procédé selon la revendication 3, dans lequel le modèle de perception de bruit comprend en outre l'application d'un modèle de transfert de bruit (710) au modèle de perception de bruit, dans lequel le modèle de transfert de bruit comprend des données pré-étalonnées relatives au transfert de bruit externe dans l'ensemble (20) haut-parleur audio.

5. Procédé selon l'une quelconque des revendications précédentes, dans lequel le procédé comprend en outre le calcul de niveaux d'ajustement de puissance de signal de test pour les unes ou plusieurs tonalités de test sur la base du modèle de perception de bruit et l'application des niveaux d'ajustement de puissance de signal aux une ou plusieurs tonalités de test.

6. Procédé selon l'une quelconque des revendications précédentes, dans lequel le procédé comprend en outre le calcul de niveaux d'ajustement de résultat de test pour le nombre de fréquences de tonalités de test sur la base du modèle de perception de bruit et l'application des niveaux d'ajustement de résultat de test au résultat de test.

7. Procédé selon l'une quelconque des revendications précédentes, dans lequel l'évaluation (510) du niveau de bruit comprend en outre,

   la réception (610) d'un signal sonore,
   la division (620) du signal sonore en une ou plusieurs trames,
   la réduction (630) des fréquences fausses et des sons transitoires dans les unes ou plusieurs trames en utilisant des techniques de fenêtrage, de recouvrement et d'ajout,
   la transformation (640) d'une ou plusieurs trames en un domaine de fréquence à l'aide d'une transformée de Fourier rapide.

8. Procédé selon l'une quelconque des revendications précédentes, dans lequel la détermination (520) du modèle de perception de bruit comprend en outre l'application (730) de données d'étalonnage de haut-parleur audio prédéterminées comprenant une fonction de transfert de fréquence du haut-parleur audio (180).

9. Ensemble (20) haut-parleur audio configuré pour être raccordé de manière fonctionnelle à une unité d'interface (1030) d'un dispositif informatique (10) pour réaliser un test audio compensé conformément au procédé selon l'une quelconque des revendications 1 à 8, dans lequel le dispositif informatique (10) comprend en outre une première unité de commande (1010) et un premier microphone (1020), dans lequel l'ensemble (20) haut-parleur audio comprend au moins un haut-parleur audio (180), un second microphone (920) et une seconde unité de commande (910) raccordée de manière fonctionnelle audit au moins un haut-parleur audio (180), dans lequel la seconde unité de commande (910) est configurée pour commander à l'au moins un haut-parleur audio (180) de présenter une ou plusieurs tonalités de test d'un nombre de fréquences de tonalités de test, et dans lequel la seconde unité de commande (910) est en outre configurée pour, sur la base d'instructions reçues de la première unité de commande (1010), réaliser :

de manière sensiblement continue pendant la durée du test auditif, l'évaluation d'un niveau de bruit, perçu par les premier et/ou second microphones (920, 1020) et le calcul d'un spectre de puissance du bruit sur la base du niveau de bruit évalué, le spectre de puissance du bruit comprenant le niveau de bruit d'un nombre de plages de fréquence,

la détermination d'un modèle de perception de bruit sur la base dudit niveau de bruit et d'un masquage hors fréquence (122) entre les une ou plusieurs fréquences de tonalités de test et le spectre de puissance du bruit pour chacune du nombre de plages de fréquence, dans lequel le modèle de perception de bruit est mis à jour de manière sensiblement continue pendant la durée du test auditif par évaluation sensiblement continue du niveau de bruit, ou

la compensation du résultat de test desdites une ou plusieurs tonalités de test à l'aide du modèle de perception de bruit.

10. Ensemble (20) haut-parleur audio selon la revendication 9, dans lequel la seconde unité de commande (910) est en outre configurée pour, sur la base des instructions reçues de la première unité de commande (1010) :

acquérir des données d'étalonnage de haut-parleur audio prédéterminées comprenant une fonction de transfert de fréquence du haut-parleur audio (180),

compenser (820) au moins l'un du résultat de test ou desdites une ou plusieurs tonalités de test à l'aide des données d'étalonnage de haut-parleur audio prédéterminées.

11. Dispositif informatique (10) configuré pour être raccordé de manière fonctionnelle à un ensemble (20) haut-parleur audio pour réaliser un test auditif compensé conformément au procédé selon l'une quelconque des revendications 1 à 8, l'ensemble (20) haut-parleur audio comprenant au moins un haut-parleur audio (180), un second microphone (920) et une seconde unité de commande (910), dans lequel le dispositif informatique (10) comprend :

une unité interface (1030), un premier microphone (1020) et une première unité de commande (1010) ;

dans lequel la première unité de commande (1010) est configurée pour ordonner à la seconde unité de commande (910) de, par l'intermédiaire des un ou plusieurs haut-parleurs audios (180), présenter une ou plusieurs tonalités de test d'un nombre de fréquences de tonalités de test, et

dans lequel la première unité de commande (1010) est en outre configurée pour réaliser :

de manière sensiblement continue pendant la durée du test auditif, l'évaluation d'un niveau de bruit, perçu par les premier et/ou second microphones (920, 1020),

la détermination d'un modèle de perception de bruit sur la base dudit niveau de bruit et d'un masquage hors fréquence (122) entre les une ou plusieurs fréquences de tonalités de test et le spectre de puissance du bruit pour chacune du nombre de plages de fréquence, dans lequel le modèle de perception de bruit est mis à jour de manière sensiblement continue pendant la durée du test auditif par évaluation sensiblement continue du niveau de bruit, ou

la compensation du résultat de test et desdites une ou plusieurs tonalités de test à l'aide du modèle de perception de bruit.

12. Dispositif informatique (10) selon la revendication 11, dans lequel le dispositif informatique (10) est en outre configuré pour :

acquérir des données d'étalonnage de haut-parleur audio prédéterminées comprenant une fonction de transfert de fréquence du haut-parleur audio (180),

compenser (820) au moins l'un du résultat de test ou desdites une ou plusieurs tonalités de test à l'aide des données d'étalonnage de haut-parleur audio prédéterminées.

13. Système (100) de réalisation d'un test auditif compensé, le test auditif étant réalisé en permettant à un utilisateur d'indiquer une perception d'une ou plusieurs tonalités de test d'un nombre de fréquences de tonalités de test, le système (100) comprenant :

un dispositif informatique (10) comprenant une unité interface (1030), un premier microphone (1020) et une première unité de commande (1010),

un ensemble (20) haut-parleur audio comprenant au moins un haut-parleur audio (180), un second microphone (920) et une seconde unité de commande (910), dans lequel

la première unité de commande (1010) est raccordée de manière fonctionnelle à la seconde unité de commande (910) et dans lequel l'ensemble (20) haut-parleur audio est l'ensemble (20) haut-parleur audio selon l'une quelconque des revendications 9 ou 10 ou le dispositif informatique (10) est le dispositif informatique (10) selon l'une quelconque des revendications 11 ou 12.

Fig. 1a

100

110        120        130

Noise evaluation module

Noise perception model determination module

Test tone compensation module

170

Speaker characteristics profiling module

150

Test result compensation module

160

140    Test tone generation module

180

Fig. 1b

110

| 111 | 112 | 113 |
|---|---|---|
| Analogue to digital conversion | Frame-by-frame buffers | Windowing, overlap and add |

| 114 | 115 | 116 |
|---|---|---|
| Fast Fourier Transform | Averaging and smoothing | Spectral power calculation |

Fig. 2

120

| 121 | 122 | 123 |
|---|---|---|
| Noise leakage calculation | Frequency masking calculation | Noise perception model calculation |

Fig. 3

Fig. 4a

Fig. 4b

Fig. 5

510

610                    620                    630

```
     ┌──────────────┐    ┌──────────────┐    ┌──────────────┐
──→  │              │    │              │    │              │
     │  Receiving   │ ─→ │   Dividing   │ ─→ │   Reducing   │ ──┐
     │              │    │              │    │              │   │
     └──────────────┘    └──────────────┘    └──────────────┘   │
                                                                 │
┌────────────────────────────────────────────────────────────────┘
│
```

640                    650                    660

```
│    ┌──────────────┐    ┌ ─ ─ ─ ─ ─ ─ ┐    ┌──────────────┐
└──→ │              │    │              │    │              │
     │ Transforming │ ─→   Averageing    ─→ │ Calculating  │ ──→
     │              │    │              │    │              │
     └──────────────┘    └ ─ ─ ─ ─ ─ ─ ┘    └──────────────┘
```

## Fig. 6

520

710                    720                    730

```
     ┌ ─ ─ ─ ─ ─ ─ ┐    ┌ ─ ─ ─ ─ ─ ─ ┐    ┌──────────────┐
     │ Applying a noise │    Off frequency     │    Noise perception │
       transfer model  ─→    masking        ─→   model determining  ─→
     │                │    │  determining │    │              │
     └ ─ ─ ─↑─ ─ ─ ─ ┘    └ ─ ─ ─ ─ ─ ─ ┘    └──────────────┘
           │
     ┌ ─ ─ ─ ─ ─ ─ ┐
     │ Applying audio │        730
──→    speaker calibration
     │     data     │
     └ ─ ─ ─ ─ ─ ─ ┘
```

## Fig. 7

820

Compensating

810

Acquiring

AND / OR

Test tone

Test result

Fig. 8

20

910

920

180

Fig. 9

Fig. 10

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006215844 A
- CN 101862195 A
- US 2015358745 A
- US 2014309549 A
- US 6160893 A
- US 2018098720 A

**Non-patent literature cited in the description**

- **YOUNGMIN et al.** *Smartphone-Based Hearing Screening in Noisy Environments,* 2014